# EUROPÄISCHE PATENTSCHRIFT

(11) **EP 0 825 441 B1**
(45) Veröffentlichungstag und Bekanntmachung des Hinweises auf die Patenterteilung: **26.03.2003**
(21) Anmeldenummer: 97110263.7
(22) Anmeldetag: 24.06.1997
(51) Int. Cl.: G01N 33/543, G01N 33/566, G01N 33/569

(54) **Sensitivitätssteigerung bei der immunchemischen Bestimmung eines Analyten**
Sensitivity enhancement in the immunochemical determination of an analyte
Augmentation de sensibilité en détermination immunochimique d'un analyte

(30) Priorität: 22.07.1996 DE 19629444
(43) Veröffentlichungstag der Anmeldung: 25.02.1998
(73) Patentinhaber: Dade Behring Marburg GmbH, 35001 Marburg (DE)
(72) Erfinder: Brust, Stefan, Dr., 35041 Marburg (DE)

(56) Entgegenhaltungen:
- EP-A- 0 572 217
- EP-A- 0 572 845

## Beschreibung

Die vorliegende Erfindung betrifft ein Verfahren zur Bestimmung eines Analyten, worin eine den zu bestimmenden Analyten gegebenenfalls enthaltende Probe mit einem ersten an einer festen Phase immobilisierten Rezeptor R1, welcher eine Bindungsaffinität zum Analyten besitzt, in Kontakt gebracht wird und ein Rezeptor R2, der ebenfalls eine Bindungsaffinität zum Analyten besitzt, zugesetzt wird und worin weiterhin die entstandenen Immunkomplexe mit einem Bindefaktor in Kontakt gebracht werden, der eine Bindungsaffinität zum Analyten und zu einem an derselben oder einer zweiten festen Phase immobilisierten Rezeptor R3 besitzt und worin die Menge des an der ersten festen Phase oder, falls vorhanden, der zweiten festen Phase gebundenen Analyten in geeigneter Weise detektiert wird.

Übliche immunologische Verfahren zur Diagnose von Erkrankungen, die mit der Bildung von spezifischen Antikörpern gegen krankheitsauslösende Agenzien, wie Viren, Bakterien, Allergene, Autoantigene oder gegen bestimmte Arzneimittel einhergehen, beruhen auf der Fähigkeit der Antikörper zur Komplexbildung mit antigenen Strukturen des Auslösers.

Bei einigen dieser Verfahren, die allgemeinen als heterogene Immunoassays bezeichnet werden, wird eine auf den Gehalt an beispielsweise spezifischem Antikörper (Analytantikörper) zu prüfende Probe mit antigenen Strukturen eines Krankheitsauslösers in Kontakt gebracht, wobei diese antigenen Strukturen an geeigneten, bekannten Trägermaterialien immobilisiert sind. In der Probe enthaltene Analytantikörper werden als Immunkomplex an die an den Trägermaterialien immobilisierten antigenen Strukturen des Krankheitsauslösers gebunden und nachgewiesen. Zum Nachweis können Nachweisantikörper bzw. andere spezifische Rezeptoren, wie beispielsweise Protein A, die zur Komplexbildung mit den Analytantikörpern der Probe befähigt sind, verwendet werden. Das Nachweisreagenz trägt in der Regel eine Markierung, welche die Menge des gebundenen Antikörpers meßtechnisch erfaßbar macht. Gebräuchliche Markierungen sind beispielsweise: radioaktive Isotope, Enzyme, fluoreszierende, phosphoreszierende oder lumineszierende Substanzen, Substanzen mit stabilen ungepaarten Elektronen, Erythrozyten, Latexpartikel, Magnetpartikel und Metallsole.

Bei diesen Verfahren sind sowohl einstufige wie auch mehrstufige Nachweismethoden bekannt. Jeder Verfahrensschritt wird üblicherweise mit einem Trennprozess (Waschschritt) beendet. Die sehr einfach durchzuführende Technik der Einschritt-Methode bei heterogenen Immunoassays ist allerdings nicht zum Nachweis aller Krankheitsmarker geeignet. Oft müssen aus technischen Gründen zwei- oder mehrstufige Verfahrensschritte angewandt werden. Bekannt sind auch mehrstufige Verfahren, die als Immunkomplex-Transfer-Enzymimmunoassays bezeichnet werden (S. Hashida et al., Journal of Clinical Laboratory Analysis 8:86-95 (1994)). Dabei wird der gesamte aus Festphasenantigen, spezifischem Antikörper und markiertem Konjugatantigen bestehende Immunkomplex von der Festphase abgelöst. Nach Überpipettieren auf eine Antikörper-bindende Festphase wird der gesamte Immunkomplex fixiert und detektiert.

Diese Methoden sind sehr spezifisch, haben allerdings den Nachteil, daß die nachzuweisenden Krankheitsauslöser oder gegen sie gerichtete Antikörper, welche im ersten Schritt mit dem immobiliserten, spezifischen Rezeptor einen Komplex eingegangen sind, in den darauffolgenden Reaktionsschritten in einer dem Fachmann bekannten Rückreaktion sich zum Teil wieder aus dem Komplex lösen können und sich somit der Nachweisreaktion entziehen, wodurch u.a. die Sensitivität stark eingeschränkt wird. Die diagnostische Leistungsfähigkeit solcher mehrstufiger Verfahren wird insbesondere dann besonders eingeschränkt, wenn die Rückreaktionsrate zwischen immobilisiertem Rezeptor und dem nachzuweisenden Agens hoch ist. Dies ist z.B. bei niederaffinen Antikörper gegen Krankheitsauslöser oder Arzneimittel der Fall. Dem Fachmann bekannt sind diese Effekte besonders bei Verfahren zum Nachweis von häufig mutierenden Krankheitsauslösern oder Krankheitsmarkern, welche mit dem immobiliserten spezifischen Rezeptor nach Mutation eine geringe Wechselwirkung zeigen.

Bereits in der EP 0 572 845 wurde offenbart, daß die Rückreaktionsrate durch Zusatz eines weiteren Bindefaktors gegen Strukturmerkmale des nachzuweisenden Agens erheblich reduziert wird. Dieser weitere Bindefaktor muß mehr als eine bindungsfähige Stelle für das nachzuweisenden Agens aufweisen und darf den immunchemischen Nachweis des Agens nicht stören. Trotz deutlich reduzierter Rückreaktion ermöglicht aber auch diese Methode nicht, spezielle niederaffine Antikörper gegen Krankheitsauslöser oder Arneimittel sicher und mit hoher Sensitivität nachzuweisen.

Der vorliegenden Erfindung lag daher die Aufgabe zu Grunde, Reagenzien zu finden, welche diese Nachteile nicht aufweisen.

Es wurde festgestellt, daß nach erfolgter Inkubation des im ersten Inkubationsschritt bereits gebildeten Immunkomplexes aus immobilisiertem spezifischen Bindungspartner R1 und damit bioaffin wechselwirkenden Analyten mit einem gegen diesen Analyten gerichteten spezifischen Bindungspartner R2, welcher in der Regel markiert ist, ein hoher Anteil an Immunkomplex aus Analyt und R2 in Lösung zu finden ist. Dieser Anteil läßt sich zwar, wie in der EP-0 572 845 beschrieben, durch Zugabe eines Rezeptors, welcher mehr als eine bindungsfähige Stelle für den Analyten besitzt, durch Fixierung an die Festphase reduzieren, überschreitet aber bei einigen Proben immer noch den Anteil des immobiliserten Immunkomplexes.

Überraschenderweise wurde jedoch festgestellt, daß durch Zugabe eines Bindefaktors und Immobilisieren eines weiteren Rezeptors R3 an die Festphase, wobei der Bindefaktor eine Affinität zum Analyten und zum Rezeptor R3 besitzt, der Anteil an nicht immobilisiertem Immunkomplex aus Analyt, R2 und Bindefaktor weiter vermindert wird. Da R2 in der Regel die Markierung trägt, wird die Signalausbeute deutlich gesteigert und somit die Sensitivität des Assays für den Analyten erhöht. Es wurde darüberhinaus festgestellt, daß nach Überführen des nicht immobiliserten Anteils des Immunkomplexes aus Analyt, R2 und Bindefaktor auf eine im wesentlichen mit dem Rezeptor R3 beschichtete zweite Festphase, ein sehr sensitiver und spezifischer Assay durchgeführt werden kann.

Im Gegensatz zu dem "Bindefaktor" genannten Rezeptor gemäß der EP-0 572 845, benötigt der Bindefaktor gemäß vorliegender Erfindung nur eine bindungsfähige Stelle für den Analyten. Ein Analyt im Sinne vorliegender Erfindung kann sowohl ein Antikörper, der beispielsweise durch einen Krankheitsauslöser induziert wurde, als auch ein Antigen, wie beispielsweise der Krankheitsauslöser selbst sein.

Die vorliegende Erfindung betrifft daher:
a) Verfahren zur Bestimmung eines Analyten, worin
   (1) eine den zu bestimmenden Analyten gegebenenfalls enthaltende Probe mit einem ersten an einer festen Phase immobilisierten Rezeptor R1, welcher eine Bindungsaffinität zum Analyten besitzt, in Kontakt gebracht wird und ein Rezeptor R2, der ebenfalls eine Bindungsaffinität zum Analyten besitzt, zugesetzt wird, dadurch gekennzeichnet, daß die entstandenen Immunkomplexe mit einem Bindefaktor in Kontakt gebracht werden, der eine Bindungsaffinität zum Analyten und zu einem an derselben oder einer zweiten festen Phase immobilisierten Rezeptor R3, jedoch keine Bindungsaffinität zu dem immobilisierten Rezeptor R1 besitzt und nicht markiert ist und
   (2) die Menge des an der ersten festen Phase oder, falls vorhanden, der zweiten festen Phase gebundenen Analyten in geeigneter Weise detektiert wird.
b) Verfahren gemäß a, dadurch gekennzeichnet, daß die Rezeptoren R1 und R3 an derselben festen Phase immobilisiert sind und daß R3 keine bioaffine Wechselwirkung mit dem Analyten oder R2 eingeht.
c) Verfahren gemäß a, dadurch gekennzeichnet, daß die Rezeptoren R1 und R3 an verschiedenen festen Phasen immobilisiert sind und daß die nach Zusammenbringen von Rezeptor R1, Analyt, Rezeptor R2 und Bindefaktor entstandenen, nicht an der ersten festen Phase immobilisierten Immunkomplexe mit der zweiten festen Phase in Kontakt gebracht werden.
d) Verfahren gemäß c, dadurch gekennzeichnet, daß eine die nicht immobilisierten Immunkomplexe enthaltende Lösung von der ersten festen Phase abgetrennt und zur zweiten, räumlich von der ersten getrennten festen Phase überführt wird.
e) Verfahren gemäß a, dadurch gekennzeichnet, daß der Analyt oder der Rezeptor R2 eine detektierbare Markierung trägt.
f) Verfahren gemäß e, dadurch gekennzeichnet, daß der Rezeptor R2 direkt oder indirekt markiert ist.
g) Verfahren gemäß a, dadurch gekennzeichnet, daß der Analyt ein Antigen ist.
h) Verfahren gemäß a, dadurch gekennzeichnet, daß der Analyt ein Antikörper ist.
i) Verfahren gemäß a, dadurch gekennzeichnet, daß der Bindefaktor ein Konjugat ist, das einen Antikörper oder ein Antikörperfragment und ein weiteres Strukturelement enthält, wobei der Antikörper oder das Antikörperfragment eine Bindungsaffinität zu dem einen und das weitere Strukturelement eine Bindungsaffinität zu dem anderen Element, ausgewählt aus der Gruppe der Elemente Analyt und Rezeptor R3, besitzt.
k) Verfahren gemäß i, dadurch gekennzeichnet, daß der Bindefaktor ein biotinylierter Antikörper und der Rezeptor R3 ein gegen Biotin gerichteter Antikörper ist.
l) Verfahren gemäß i, dadurch gekennzeichnet, daß der Bindefaktor ein biotinylierter Antikörper und der Rezeptor R3 Avidin oder Streptavidin ist.
m) Verfahren gemäß a zum Nachweis von Antikörpern gegen das humane Immundefizienzvirus (HIV).
n) Testkit zur Verwendung in einem Verfahren gemäß b, dadurch gekennzeichnet, daß er eine feste Phase mit den darauf immobilisierten Rezeptoren R1 und R3 sowie den Bindefaktor in solubilisierter oder solubilisierbarer Form enthält.
o) Testkit zur Verwendung in einem Verfahren gemäß c, dadurch gekennzeichnet, daß er eine erste feste Phase mit darauf immobilisiertem Rezeptor R1, eine zweite feste Phase mit darauf immobilisiertem Rezeptor R3 und den Bindefaktor in solubilisierter oder solubilisierbarer Form enthält.

Daß der Bindefaktor nicht markiert ist bedeutet im Sinne vorliegender Erfindung, daß er entweder kein oder zumindestens nicht dasselbe Label trägt, mit dem das Ausmaß der Bindung des Analyten an die erste oder zweite feste Phase bestimmt wird.

Verfahren, in denen die immobilisierten Rezeptoren R3 verwendet werden können, sind in allen ihren Ausführungsformen dem Fachmann bekannt. Wichtig ist, daß die erfindungsgemäßen Verfahren in geeigneter Form bei allen immunchemischen Verfahren, bei denen in einem ersten Schritt eine immunchemische oder vergleichbare Bindung eines Analyten an einen bevorzugterweise immobilisierten, spezifischen Rezeptor erfolgt und in einem zweiten, aber nicht notwendigenweise zeitlich getrennten Schritt ein direkter oder indirekter Nachweis erfolgt, eingesetzt werden können. Bevorzugt werden die erfindungsgemäßen Verfahren in den dem Fachmann als Sandwich-ELISAs bekannten Verfahren eingesetzt, wobei bevorzugterweise ein Enzym als Markierungssystem, bevorzugterweise mit einem chromogenen oder fluorogenen Substrat oder ein chemoluminsezentes Label als Markierungssystem verwendet wird. Die gewählte Ausführungsform beeinflußt jedoch nicht prinzipiell die Verwendungsmöglichkeiten der erfindungsgemäßen Verfahren.

Als Festphasen werden bevorzugterweise Mikrotitrationsplatten, Magnetpartikel, Latexpartikel oder matrixchemische Testelemente, wie z.B. Fasern oder Membranen enthaltende Testmodule verwendet.

Dem Fachmann ist bekannt, daß immunchemische Verfahren, wie oben beschrieben, zur gleichzeitigen Bestimmung von unterschiedlichen Analyten, wie beispielsweise HIV 1, HIV 2 oder HCV, sowie von gegen diese Analyten gerichteten Antikörpern eingesetzt werden können. Solche Ausführungsformen sind hiermit ausdrücklich eingeschlossen.

Bindefaktoren im Sinne vorliegender Erfindung sind spezifische Rezeptoren, die eine oder mehr als eine bioaffine Bindungsstelle zu R2 aufweisen. Weiterhin gehen sie eine Wechselwirkung mit dem Rezeptor R3 ein. Bindungspartner oder Komponenten der Bindefaktoren können Antikörper, Antikörperfragmente oder Konjugate enthaltend Antikörper oder Antikörperfragmente und eine weitere Komponente sein, wie beispielsweise ein Enzym. Diese Antikörper oder Antikörperfragmente bzw. Konjugate daraus können gegen den Analyten oder aber auch gegen den Analyten und R3 gerichtet sein. Der Bindefaktor kann auch aus einem Antikörper oder Antikörperfragment bestehen, welches mit einem Strukturelement gekoppelt ist, gegen das der Rezeptor R3 gerichtet ist.

Rezeptoren R3 im Sinne vorliegender Erfindung sind spezifische Bindungspartner, die eine oder mehr als eine bioaffine Bindungsstelle zum Bindefaktor aufweisen. Rezeptoren R3 oder Komponente dieses Bindungspartners können Antikörper, Antikörperfragmente oder Konjugate enthaltend Antikörper oder Antikörperfragmente und eine weitere Komponente sein, wie beispielsweise ein Enzym. Diese Antikörper oder Antikörperfragmente bzw. Konjugate daraus können gegen den Bindefaktor gerichtet sein oder aber antigene Strukturen aufweisen, die von Bindungsstellen des Bindefaktors erkannt werden. Rezeptor R3 kann auch aus einem Protein bestehen, welches mit einem Strukturelement gekoppelt ist, gegen das der Bindefaktor gerichtet ist.

Bevorzugte Kombination von Bindungspartnern zum spezifischen Nachweis von Antigenen:
- R1:: an einer festen Phase immobilisierter Antikörper, der gegen den Analyten gerichtet ist;
- Analyt:: Antigen;
- R2:: mit einem Label versehener Antikörper (=Konjugatantikörper), der gegen den Analyten gerichtet ist;
- Bindefaktor:: Antikörper, der gegen den Analyten gerichtet ist;
- R3:: an der festen Phase immobilisierter Antikörper, der gegen den Bindefaktor gerichtet ist.

Im Falle des Antigennachweises wird bevorzugterweise ein Antikörper bzw. ein Antikörperfragment als Bindefaktor verwendet, welcher(es) nicht dasselbe Epitop erkennt wie der Festphasenantikörper R1 oder der Konjugatantikörper R2.

Bevorzugte Kombination von Bindungspartnern zum Nachweis spezifischer Antikörper:
- R1:: an einer festen Phase immobilisiertes Antigen, gegen das der Analyt gerichtet ist;
- Analyt:: Antikörper;
- R2:: mit einem Label versehenes Antigen, gegen das der Analyt gerichtet ist;
- Bindefaktor:: Antikörper, der gegen den Analyten gerichtet ist;
- R3:: an der festen Phase immobilisierter Antikörper, der gegen den Bindefaktor gerichtet ist.

Besonders bevorzugte Kombination von Bindungspartnern zum Nachweis spezifischer Antikörper:
- R1:: an einer festen Phase immobilisiertes Antigen, gegen das der Analyt gerichtet ist;
- Analyt:: Antikörper;
- R2:: mit einem Label versehenes Antigen, gegen das der Analyt gerichtet ist;
- Bindefaktor:: Antikörper, der gegen den Analyten gerichtet ist und zusätzliche, nicht antikörpereigene Epitope trägt;
- R3:: an der festen Phase immobilisierter Antikörper, der gegen das zusätzliche, nicht antikörpereigene Epitop des Bindefaktors gerichtet ist.

Dem Fachmann sind Methoden zur Herstellung von Konjugaten, die aus einem Antikörper und einem zusätzlichen nicht antikörpereigenen Antigen (beispielsweise Biotin) bestehen bekannt. Solche Konjugate können, unter Erhalt der bioaffinen Funktion ihrer Ausgangsmaterialien, beispielsweise durch Verknüpfung mittels chemischer Reagenzien oder durch bioaffine Wechselwirkung hergestellt werden. Es können auch Hybridmoleküle durch chemische Synthese, die Hybridoma-Technik oder gentechnologische Methoden erzeugt werden.

Das erfindungsgemäße Reagenz kann in einer Vielzahl von Verfahren der Human- und Veterinärdiagnostik eingesetzt werden. Als Beispiele sollen angeführt werden: zwei- oder mehrstufige Teste zu Nachweis von Antikörpern verschiedener Immunglobulin-Klassen gegen Strukturmerkmale von Viren (z.B.

Viren der Hepatitis A, B und C, sowie verschiedener HIV-Typen) und Nachweisverfahren von bakteriellen und parasitären Erregern sowie von allergischen Erkrankungen. Weitere Beispiele sind der Nachweis von Krankheitserregern, wie Viren (z.B. Hepatitis B-Virus), Bakterien, Parasiten und Allergenen, wie auch von Markern von Krankheiten (z.B. Tumormarkern) in einund mehrstufigen Nachweisverfahren.

Die Erfindung wird außerdem durch die folgenden Beispiele erläutert, ohne sie jedoch darauf einzuschränken.

### Beispiel 1

### a) Herstellung der Festphase

Mikrotitrationsplatten Typ B (Fa. Nunc, Roskilde, Dänemark) werden mit 100 µl je Vertiefung Beschichtungslösung (600 µg/ml rekombinantes gp41 [Behringwerke AG, Marburg, BRD] und 10 mg/ml monoklonalem Antikörper gegen Biotin [Behringwerke AG, Marburg, BRD] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß für 1 Jahr stabil.

### b) Herstellung des Konjugats

10 mg HIV 1-gp41-Peptid (IAF Biochem, Laval, Kanada) werden in 1 ml Eisessig/Wasser (50:50, v/v) gelöst. Nach Neutralisieren mit 5N Natronlauge wird mit einem 10fach molaren Überschuß an N-γ-Maleimidobutyrylsuccinimid versetzt und 1 Stunde bei Raumtemperatur inkubiert. Das nicht umgesetzte heterobifunktionelle Reagenz wird durch Gelfiltration (Sephadex G-25) mit 100 mM Natriumphosphat, 5 mM Nitrilotriessigsäure, pH 6.0 abgetrennt.
10 mg Meerrettich-Peroxidase (Boehringer Mannheim, Mannheim, BRD) werden in 10 ml 10 mM Natriumphosphat, 100 mM Kochsalz, pH 8.0 mit 100fach molarem Überschuß an 2-Iminothiolan 1 Stunde bei Raumtemperatur inkubiert. Anschließend wird freies Modifierungsreagenz durch Gelfiltration (Sephadex G-25) mit 100 mM Natriumphosphat, 5 mM Nitrilotriessigsäure, pH 6.0 entfernt. Beide Eluate (SH-aktivierte Peroxidase und Maleimid-modifizierte HIV 1-Peptid) werden vereinigt und über Nacht bei Raumtemperatur inkubiert. Nach Abstoppen mit 1/10 Vol 100 mM N-Ethyl-Maleimid wird das Konjugat durch Gelfiltration (Sephadex G-25) von nicht abreagiertem HIV 1-Peptid gereinigt, Nach Ankonzentrieren (2 mg/ml) wird das Konjugat bei -20°C gelagert.

### c) 2-Schritt-Enzymimmunoassay zum Nachweis von HIV 1-Antikörpern

Ein Enzymimmunassay zum Nachweis von Anti-HIV 1 wird wie folgt durchgeführt: In die Vertiefungen der nach Beispiel 1a hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 100 µl des nach Beispiel 1b hergestellten Konjugats (1:1000 in 0.1 M Tris/HCl, 1 % Albumin, 2% Pluronic F64, pH 8.1) einpipettiert. Mit 4 weiteren Waschschritten wird die 30-minütige Inkubation (+37°C) beendet. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt.

### d) Herstellung des biotinylierten Antikörpers

10 mg Monoklonaler Antikörper gegen humanes Immunglobulin G [Behringwerke AG, Marburg, BRD] werden in 10 ml 10 mM Natriumphosphat, 100 mM Kochsalz, pH 8.0 mit 10fach molarem Überschuß an N-Hydroxysuccinimid-X-Biotin in 10 mM Natriumphosphat, 100 mM Kochsalz, pH 8.0 für 1 Stunde bei Raumtemperatur inkubiert. Anschließend wird freies Modifierungsreagenz durch Gelfiltration (Sephadex G-25) mit 100 mM Tris, 5 mM Nitrilotriessigsäure, pH 7.0 entfernt.

### e) Anwendung des erfindungsgemäßen Reagenzes

Anti-HIV 1 positive und anti-HIV negative Seren werden im Enzymimmunoassay nach Beispiel 1c untersucht (Referenzsystem I). Anti-HIV 1 positive Proben mit ungewöhnlich niedriger Reaktivität werden zusätzlich im erfindungsgemäßen Assaysystem untersucht (Erfindungsgemäßes System I). Dazu wird der Konjugatlösung nach Beispiel 1c zusätzlich ein nach Beispiel 1d hergestellter biotinylierter Anti-human IgG-Antikörper (1 µg/ml) zugesetzt.

Als weiteres Referenzsystem wird dieser Anti-human IgG-Antikörper ohne Biotin-Fragment in einer Konzentration von 1 µg/ml dem Konjugat von Beispiel 1c zugegeben (Referenzsystem II). Die Resultate (Extinktionseinheiten) der Untersuchung finden sich in der Tabelle 1.

**Tabelle 1:**

| **Proben- Bezeichnung** | **Anti-HIV Status** | **Verdünnung** | **Bemerkung** | **Referenz- system I** | **Referenz- system II** | **Erfindungs- gemäßes System I** |
|---|---|---|---|---|---|---|
| **Negative Kontrolle** | Negativ | nativ | | 0,053 | 0,039 | 0,042 |
| **Positive Kontrolle** | Positiv | 1:800 | hochaffin | 2,076 | 1,875 | 1,964 |
| **9111/39** | Positiv | 1:20 | niederaffin | 1,059 | 1,827 | 2,276 |
| **9111/41** | Positiv | 1:10 | niederaffin | 0,476 | 0,873 | 1,659 |
| **9111/54** | Positiv | 1:20 | niederaffin | 1,049 | 1,915 | >2,500 |
| **9111/53** | Positiv | 1:10 | niederaffin | 0,407 | 0,952 | 1,947 |
| **9304/11** | Positiv | 1:500 | hochaffin | 1,049 | 0,862 | 1,183 |
| **BS 5** | Negativ | nativ | | 0,084 | 0,42 | 0,063 |
| **BS 54** | Negativ | nativ | | 0,059 | 0,054 | 0,054 |
| **BS 13** | Negativ | nativ | | 0,061 | 0,048 | 0,070 |
| **BS 15** | Negativ | nativ | | 0,071 | 0,039 | 0,053 |

Deutliche Unterschiede in der Signalbildung bei den 3 Testsystemen ist insbesondere bei niederaffinen Proben (z.B. 9111/53) zu erkennen. Die Sensitivität bezüglich dieser Proben wird um den Faktor 4 gegenüber dem Referenzsystem I und das Doppelte gegenüber dem Referenzsystem II gesteigert.

Proben mit hoher Affinität zum Beschichtungsantigen, welche auch im Referenzsystem I bereits in hoher Verdünnung sicher erkannt werden, sowie Anti-HIV negative Seren, erfahren im erfindungsgemäßen System I keinen Signalzuwachs.

### Beispiel 2

### a) Herstellung der Festphase

Mikrotitrationsplatten (Fa. Nunc, Roskilde, Dänemark, TypB) werden mit 100 µl je Vertiefung Beschichtungslösung (10 mg/ml Antikörper vom Kaninchen gegen Maus-Immunglobulin [Behringwerke AG, Marburg, BRD] in 50 mM Natriumcarbonat-Puffer, pH 9.5) für 24 Stunden bei 4°C inkubiert. Die Vertiefungen der Mikrotitrationsplatten werden dann dreimal mit je 300 µl Waschlösung (50 mM Tris, 0.1% Tween 20, pH 7.2) gewaschen. Die über Kieselgel getrockneten Mikrotitrationsplatten sind unter Luftausschluß für 1 Jahr stabil.

### b) Transfer-Enzymimmunoassay zum Nachweis von HIV 1-Antikörpern

Ein Transfer-Enzymimmunassay zum Nachweis von Anti-HIV 1 wird wie folgt durchgeführt:
In die Vertiefungen der nach Beispiel 1a hergestellten Mikrotitrationsplatten werden 25 µl Probenpuffer (0.3 M Tris/HCl, 1% Albumin, 2% Tween 20, pH 7.2) mit 100 µl Humanserum für 30 Minuten bei 37°C inkubiert. Nach 4maligem Waschen mit 50 mM PBS, 0.1% Tween 20 werden 100 µl des nach Beispiel 1b hergestellten Konjugats (1:1000 in 0.1 M Tris/HCl, 1 % Albumin, 2% Pluronic F64, pH 8.1) einpipettiert, das durch zusätzliche Zugabe von 1 µg/ml monoklonalem Antikörper gegen humanes Immunglobulin aufgestockt wurde. Nach 30minütiger Inkubation (+37°C) wird die Lösung der Konjugatphase (100 µl) in die Vertiefungen der nach Beispiel 2a hergestellten Mikrotitrationsplatten überpipettiert (erfindungsgemäßes System II). Die Antigen-beschichteten Testplatten werden wie oben beschrieben weiter bearbeitet und ausgewertet (Referenzsystem III). Die Mikrotitrationsplatten nach Beispiel 2a die mit Antikörper beschicht wurden, und nun die überpipettierten Immunkomplexe enthalten, werden nach weiteren 30 Minuten Inkubation bei 37°C viermal gewaschen. Die gebundene Peroxidase-Aktivität, die direkt mit der Anzahl der gebundenen Anti-HIV 1-Antikörper korreliert, wird durch Zugabe von H₂O₂/ Tetramethylbenzidin bestimmt. Der Substratumsatz wird nach 30 Minuten bei Raumtemperatur durch Zugabe von 0.5 M Schwefelsäure gestoppt. Die Extinktion wird bei 450 nm festgestellt. Die Resultate (Extinktionseinheiten) der Untersuchung finden sich in der Tabelle 2.

**Tabelle 2**

| **Proben- Bezeichnung** | **Anti-HIV Status** | **Verdünnung** | **Bemerkung** | **Referenzsystem III** | **Erfindungsgemäßes System II** |
|---|---|---|---|---|---|
| **Negative Kontrolle** | Negativ | nativ | | 0,104 | 0,106 |
| **Positive Kontrolle** | Positiv | 1:800 | hochaffin | 1,868 | 1,274 |
| **9111/39** | Positiv | 1:20 | niederaffin | 1,424 | >2,500 |
| **9111/41** | Positiv | 1:10 | niederaffin | 0,835 | 2,335 |
| **9111/54** | Positiv | 1:20 | niederaffin | 1,679 | >2,500 |
| **9111/53** | Positiv | 1:10 | niederaffin | 0,954 | >2,500 |
| **9304/11** | Positiv | 1:500 | hochaffin | 0,868 | 1,334 |
| **BS 5** | Negativ | nativ | | 0,157 | 0,126 |
| **BS 54** | Negativ | nativ | | 0,134 | 0,100 |
| **BS 13** | Negativ | nativ | | 0,128 | 0,099 |
| **BS 15** | Negativ | nativ | | 0,147 | 0,107 |

Deutlich ist der Signalunterschied zwischen dem Referenzsystem III und den erfindungsgemäßen System II wiederum bei den niederaffinen Proben zu erkennen. Im erfindungsgemäßen System II wird zum Teil die doppelte Sensitivität erzielt. Proben mit hoher Affinität zum Beschichtungsantigen, welche auch im Referenzsystem III bereits in hoher Verdünnung sicher erkannt werden, sowie Anti-HIV negative Seren, erfahren im erfindungsgemäßen System II keinen Signalzuwachs.

## Patentansprüche

1. Verfahren zur Bestimmung eines Analyten, worin
(1) eine den zu bestimmenden Analyten gegebenenfalls enthaltende Probe mit einem ersten an einer festen Phase immobilisierten Rezeptor R1, welcher eine Bindungsaffinität zum Analyten besitzt, in Kontakt gebracht wird und ein Rezeptor R2, der ebenfalls eine Bindungsaffinität zum Analyten besitzt, zugesetzt wird, **dadurch gekennzeichnet, daß** die entstandenen Immunkomplexe mit einem Bindefaktor in Kontakt gebracht werden, der eine Bindungsaffinität zum Analyten und zu einem an derselben oder einer zweiten festen Phase immobilisierten Rezeptor R3, jedoch keine Bindungsaffinität zu dem immobilisierten Rezeptor R1 besitzt und nicht markiert ist besitzt und
(2) die Menge des an der ersten festen Phase oder, falls vorhanden, der zweiten festen Phase gebundenen Analyten in geeigneter Weise detektiert wird.

2. Verfahren gemäß Anspruch 1, **dadurch gekennzeichnet, daß** die Rezeptoren R1 und R3 an derselben festen Phase immobilisiert sind und daß R3 keine bioaffine Wechselwirkung mit dem Analyten oder R2 eingeht.

3. Verfahren gemäß Anspruch 1, **dadurch gekennzeichnet, daß** die Rezeptoren R1 und R3 an verschiedenen festen Phasen immobilisiert sind und daß die nach Zusammenbringen von Rezeptor R1, Analyt, Rezeptor R2 und Bindefaktor entstandenen, nicht an der ersten festen Phase immobilisierten Immunkomplexe mit der zweiten festen Phase in Kontakt gebracht werden.

4. Verfahren gemäß Anspruch 3, **dadurch gekennzeichnet, daß** eine die nicht immobilisierten Immunkomplexe enthaltende Lösung von der ersten festen Phase abgetrennt und zur zweiten, räumlich von der ersten getrennten festen Phase überführt wird.

5. Verfahren gemäß Anspruch 1, **dadurch gekennzeichnet, daß** der Analyt oder der Rezeptor R2 eine detektierbare Markierung trägt.

6. , Verfahren gemäß Anspruch 5, **dadurch gekennzeichnet, daß** der Rezeptor R2 direkt oder indirekt markiert ist.

7. Verfahren gemäß Anspruch 1, **dadurch gekennzeichnet, daß** der Analyt ein Antigen ist.

8. Verfahren gemäß Anspruch 1, **dadurch gekennzeichnet, daß** der Analyt ein Antikörper ist.

9. Verfahren gemäß Anspruch 1, **dadurch gekennzeichnet, daß** der Bindefaktor ein Konjugat ist, das einen Antikörper oder ein Antikörperfragment und ein weiteres Strukturelement enthält, wobei der Antikörper oder das Antikörperfragment eine Bindungsaffinität zu dem einen und das weitere Strukturelement eine Bindungsaffinität zu dem anderen Element, ausgewählt aus der Gruppe der Elemente Analyt und Rezeptor R3, besitzt.

10. Verfahren gemäß Anspruch 9, **dadurch gekennzeichnet, daß** der Bindefaktor ein biotinylierter Antikörper und der Rezeptor R3 ein gegen Biotin gerichteter Antikörper ist.

11. Verfahren gemäß Anspruch 9, **dadurch gekennzeichnet, daß** der Bindefaktor ein biotinylierter Antikörper und der Rezeptor R3 Avidin oder Streptavidin ist.

12. Verfahren gemäß Anspruch 1 zum Nachweis von Antikörpern gegen das humane Immundefizienzvirus (HIV).

13. Testkit zur Verwendung in einem Verfahren gemäß Anspruch 2, **dadurch gekennzeichnet, daß** er eine feste Phase mit den darauf immobilisierten Rezeptoren R1 und R3 sowie den Bindefaktor in solubilisierter oder solubilisierbarer Form enthält.

14. Testkit zur Verwendung in einem Verfahren gemäß Anspruch 3, **dadurch gekennzeichnet, daß** er eine erste feste Phase mit darauf immobilisiertem Rezeptor R1, eine zweite feste Phase mit darauf immobilisiertem Rezeptor R3 und den Bindefaktor in solubilisierter oder solubilisierbarer Form enthält.

## Claims

1. A process for the determination of an analyte, in which
(1) a sample optionally containing the analyte to be determined is brought into contact with a first receptor R1, which has a binding affinity for the analyte, immobilized on a solid phase, and a receptor R2, which likewise has a binding affinity for the analyte, is added, which comprises bringing the resulting immune complexes into contact with a binding factor which has a binding affinity for the analyte and for a receptor R3 immobilized on the same or a second solid phase, but has no binding affinity for the immobilized receptor R1 and is not labeled and
(2) the amount of the analyte bound to the first solid phase or, if present, to the second solid phase is detected in a suitable manner.

2. The process as claimed in claim 1, wherein the receptors R1 and R3 are immobilized on the same solid phase and wherein R3 undergoes no bioaffinity interaction with the analyte or R2.

3. The process as claimed in claim 1, wherein the receptors R1 and R3 are immobilized on various solid phases and wherein the immune complexes not immobilized on the first solid phase, resulting after bringing together receptor R1, analyte, receptor R2 and binding factor, are brought into contact with the second solid phase.

4. The process as claimed in claim 3, wherein a solution containing the nonimmobilized immune complexes is separated off from the first solid phase and transferred to the second solid phase, which is spatially separate from the first solid phase.

5. The process as claimed in claim 1, wherein the analyte or the receptor R2 carries a detectable label.

6. The process as claimed in claim 5, wherein the receptor R2 is directly or indirectly labeled.

7. The process as claimed in claim 1, wherein the analyte is an antigen.

8. The process as claimed in claim 1, wherein the analyte is an antibody.

9. The process as claimed in claim 1, wherein the binding factor is a conjugate which contains an antibody or an antibody fragment and a further structural element, the antibody or the antibody fragment having a binding affinity for one element selected from the group of the elements analyte and receptor R3 and the further structural element having a binding affinity for the other element.

10. The process as claimed in claim 9, wherein the binding factor is a biotinylated antibody and the receptor R3 is an antibody directed against biotin.

11. The process as claimed in claim 9, wherein the binding factor is a biotinylated antibody and the receptor R3 is avidin or streptavidin.

12. The process as claimed in claim 1 for the detection of antibodies against human immunodeficiency virus (HIV).

13. A test kit for use in a process as claimed in claim 2, which comprises a solid phase having the receptors R1 and R3 immobilized thereon, and the binding factor in solubilized or solubilizable form.

14. A test kit for use in a process as claimed in claim 3, which comprises a first solid phase having a receptor R1 immobilized thereon, a second solid phase having a receptor R3 immobilized thereon and the binding factor in solubilized or solubilizable form.

## Revendications

1. Procédé pour la détermination d'un analyte, dans lequel
(1) on met l'échantillon contenant éventuellement l'analyte à déterminer en contact avec un premier récepteur R1 immobilisé sur une phase solide, lequel possède une affinité de liaison avec l'analyte, et on ajoute un récepteur R2, qui possède également une affinité de liaison avec l'analyte, **caractérisé en ce qu'**on met les complexes immuns résultants en contact avec un facteur de liaison qui possède une affinité de liaison avec l'analyte et avec un récepteur R3 immobilisé sur la même phase solide ou sur une seconde phase solide, mais ne possède aucune affinité de liaison avec le récepteur R1 immobilisé et n'est pas marqué et
(2) on détecte de façon appropriée la quantité de l'analyte fixée à la première phase solide ou, si elle est présente, à la seconde phase solide.

2. Procédé selon la revendication 1, **caractérisé en ce que** les récepteurs R1 et R3 sont immobilisés sur la même phase solide et **en ce que** R3 ne participe à aucune interaction de bioaffinité avec l'analyte ou R2.

3. Procédé selon la revendication 1, **caractérisé en ce que** les récepteurs R1 et R3 sont immobilisés sur des phases solides différentes et **en ce que** les complexes immuns non immobilisés sur la première phase solide, formés après mise en contact du récepteur R1, de l'analyte, du récepteur R2 et du facteur de liaison, sont mis en contact avec la seconde phase solide.

4. Procédé selon la revendication 3, **caractérisé en ce qu'**une solution contenant les complexes immuns non immobilisés est séparée de la première phase solide et transférée à la seconde phase solide séparée spatialement de la première.

5. Procédé selon la revendication 1, **caractérisé en ce que** l'analyte ou le récepteur R2 porte un marqueur détectable.

6. Procédé selon la revendication 5, **caractérisé en ce que** le récepteur R2 est marqué directement ou indirectement.

7. Procédé selon la revendication 1, **caractérisé en ce que** l'analyte est un antigène.

8. Procédé selon la revendication 1, **caractérisé en ce que** l'analyte est un anticorps.

9. Procédé selon la revendication 1, **caractérisé en ce que** le facteur de liaison est un conjugué qui contient un anticorps ou un fragment d'anticorps et un autre élément structural, l'anticorps ou le fragment d'anticorps possédant une affinité de liaison avec un élément et l'autre élément structural possédant une affinité de liaison avec l'autre élément, choisi dans le groupe des éléments analyte et récepteur R3.

10. Procédé selon la revendication 9, **caractérisé en ce que** le facteur de liaison est un anticorps biotinylé et le récepteur R3 est un anticorps dirigé contre la biotine.

11. Procédé selon la revendication 9, **caractérisé en ce que** le facteur de liaison est un anticorps biotinylé et le récepteur R3 est l'avidine ou la streptavidine.

12. Procédé selon la revendication 1, pour la détection d'anticorps dirigés contre le virus de l'immunodéficience humaine (VIH).

13. Nécessaire d'essai pour utilisation dans un procédé selon la revendication 2, **caractérisé en ce qu'**il contient une première phase solide comportant les récepteurs R1 et R3 immobilisés sur celle-ci, ainsi que le facteur de liaison sous forme solubilisée ou susceptible d'être solubilisée.

14. Nécessaire d'essai pour utilisation dans un procédé selon la revendication 3, **caractérisé en ce qu'**il contient une première phase solide comportant le récepteur R1 immobilisé sur celle-ci, une seconde phase solide comportant le récepteur R3 immobilisé sur celle-ci et le facteur de liaison sous forme solubilisée ou susceptible d'être solubilisée.
